# EUROPÄISCHE PATENTANMELDUNG

(11) **EP 2 537 515 A1**
(43) Veröffentlichungstag der Anmeldung: **26.12.2012**
(21) Anmeldenummer: 12173142.6
(22) Anmeldetag: 22.06.2012
(51) Int. Cl.: A61K 9/107, A61K 47/10, A61K 47/24, A61K 47/44, A61K 47/14, A61K 47/26, A61K 31/122

(54) **Diätetikum zur Behandlung mitochondrialer Dysfunktionen**

(30) Priorität: 22.06.2011 DE 102011105703
(71) Anmelder: Langhoff, Wolfgang, 50189 Elsdorf (DE)
(72) Erfinder: Langhoff, Wolfgang, 50189 Elsdorf (DE)
(74) Vertreter: von Kreisler Selting Werner

(57) **Zusammenfassung**

Die Erfindung betrifft eine flüssige, wässrige Emulsion sowie deren Verwendung als Getränk und insbesondere als Diätetikum. Darüber hinaus betrifft die vorliegende Erfindung eine pharmazeutische Zubereitung, umfassend die flüssig, wässrige Emulsion, insbesondere zur Prophylaxe und Therapie mitochondrialer Dysfunktionen. Weiterhin betrifft die Erfindung ein Pulver, welches durch Trocknung der flüssigen, wässrigen Emulsion erhältlich ist.

## Beschreibung

Die Erfindung betrifft eine flüssige, wässrige Emulsion sowie deren Verwendung als Getränk und insbesondere als Diätetikum. Darüber hinaus betrifft die vorliegende Erfindung eine pharmazeutische Zubereitung, umfassend die flüssig, wässrige Emulsion, insbesondere zur Prophylaxe und Therapie mitochondrialer Dysfunktionen. Weiterhin betrifft die Erfindung ein Pulver, welches durch Trocknung der flüssigen, wässrigen Emulsion erhältlich ist.

Mitochondriale Dysfunktionen stehen im Fokus des medizinischen Interesses.

Rolf Luft erkannte, dass eine Vielzahl mitochondrialer Dysfunktionen im Laufe des Lebens erworben werden (Luft R.: The development of mitochondrial medicine, Proc. Natl. Acad. Sci 91 (1994) (731-(734)).

Fatal ist allerdings, dass das menschliche, mitochondriale Erbgut extrem leicht verletzt werden kann. Dieses liegt völlig ungeschützt vor, da es fast nur aus Exons besteht. Jeder Angriff hat gute Chancen Schäden anzurichten. Veränderungen der mtDNA können mangels Reparatursysteme nicht wieder behoben werden.

Folgende Tabelle fasst die Besonderheiten der Mitochondrien DANN zusammen:
- Nur Exons, keine Introns
- Nackte DNA ohne Histon und keine Reparatursysteme
- Hoher Membran-Lipidgehalt (Peroxidationsgefahr)
- Gifte werden stärker angereichert (Nitrosamine)
- Spontammutationsrate 1000-fach höher
- Freie Radikale werden permanent gebildet. Bei Reperfusion werden 50% des Sauerstoff in Radikale umgewandelt, sonst nur 1-3%.

Mitochondrien und Stickstoffmonoxid (NO) Stickstoffmonoxid wird, ausgehend von L-Arginin, durch drei verschiedene NO-Synthetasen (NOS) synthetisiert. Eine davon wird durch Entzündungsprozesse aktiviert und verursacht sehr hohe NO-Spiegel.

Offensichtlich gibt es jedoch in den Mitochondrien noch eine weitere NOS (Loesch A. et al.: An ultrastructural study of NADPH-diaphorase and nitric oxide synthase in the perivascular nerves and vascular endothelium of the rat basilar artery, J. Neurocytol 23 (1994) 49-59; Lacza Z. et al.: Mitochondrial nitric oxide synthase is not eNOS, nNOS or iNOS, Free Radic. Biol. Med. 35 (2003) 1217-1228).

NO kann in sehr reaktive Moleküle umgewandelt werden, in sogenannte reaktive NO-Spezies. NO reagiert beispielsweise mit Sauerstoff, wobei NO₂ gebildet wird. Dieses ist in der Lage mit einem zweiten NO zu N₂O₃ zu reagieren. N₂O₃ kann Thiole wie z.B. Glutathion nitrosylieren. Außerdem kann es mit dem Superoxidanionenradikal (O₂^{•-}) zu Peroxynitrit (ONOO⁻) reagieren. NO und Peroxynitrit üben einen hemmenden Effekt auf die mitochondriale Atmungskette aus (Cassina A. and Radi R.: Differential inhibitory action of nitric oxide and peroxynitrite on mitochondrial electgron transport. Arch. Biochem Biophys. 328 (1996) 309-316; Brown, G.C.: Nitric oxide and mitochondrial respiration, Biochim. Biophys. Acta 1411 (1999) 351-369; Brown, G.C.: Regulation of mitochondrial respiration by nitric oxide inhibition of cytochrome c oxidase, Biochim. Biophys. Acta 1504 (2001) 46-57).

Ein schneller Effekt stellt die Hemmung des Komplexes I dar. Diese Hemmung wird hervorgerufen durch eine S-Nitrosylierung (Clementi E. et al.: Persistant inhibition of cell respiration by nitric oxide Proc. Natl. Acad. Sci U.S.A. 95 (1998) 7631-7636).

Ein Abfall des mitochondrialen Menbranpotentials stellt ein sehr frühes Ereignis in der mitochondrialen Zelltodkaskade dar (Green D.R. and Reed T.B.: Mitochondria and apoptosis, Science, 281 (1998) 1309-1312; Green D.R. and Kroemer G. The pathophysiology of mitochondrial cell death, Science 305 (2004) 626-629.)

Ein leichter Abfall des mitochondrialen Membranpotentials wird mit Apoptose in Verbindung gebracht, wohingegen ein drastischer Abfall die Nekrose begünstigt (Martinou J.C. and Green D.R.: Breaking the mitochondrial barrier, Nat. Rev. Mol. Cell. Biol. 2 (2001) 63-67).

Mitochondriale Fehlfunktion, besonders des Komplexes I, spielen eine große Rolle bei zahlreichen Erkrankungen, wie z.B. Typ-II-Diabetes, Arteriosklerose, Cardiomyopathie, Fibromyalgie, Alzheimer und Parkinson (Swerdlow R.H. et al.: Origin and fuctional consequences of the complex I defect in Parkinson disease, Ann. Neurol. 40 (1996) 663-671; Swerdlow R.H. et al.: Cybrids in Alzheimer disease a cellular model of the disease? Neurology 49 (1997) 918-925; Swerdlow R.H. and Kish S.J.: Mitochondria in Alheimer disease, Int Rev Neurobiol. 53 (2002) 341-385).

Bei der hypertrophischen Kardiomyopathie konnte P.H. Langsjoen zeigen, das die Gabe von Coenzym Q10 Atemnot und Müdigkeit positiv beeinflusst. Zudem konnte per echokardiographischen Kontrollen gezeigt werden, dass die Dicke der Herzwand zurückging (Langsjoen P.H, Willis R. Folkers K.: Treatment of hypertrophic cardiomyopathy with coenzyme Q10. 9th Int Symp. Biomed. And Clinical Aspects of Coenzymq Q10: 26 (1996)).

Kritiker warnen seit Längerem vor negativen Langzeitfolgen durch Statine. Besonders Patienten die an Herzinsuffiziens und der damit einhergehenden Verschlechterung der myokarden Pumpfuktion sind durch die Einnahme von Statinen besonders betroffen (Langsjoen P.H. Langsjoen A.M.: The clinical use of HMG-CoA-reductase inhibitors and the associated depletion of coenzyme Q10. A review of animal and human publications. Biofactors 2003; 18: 101-111). Statine hemmen die 3-Hydroxy-3-Methyl-Glutaryl-CoA Synthetase, kurz HMG-CoA Synthetase. Dadurch verlangsamt sich zwar die Cholesterin-, aber ebenso die Coenzym Q10-Synthese.

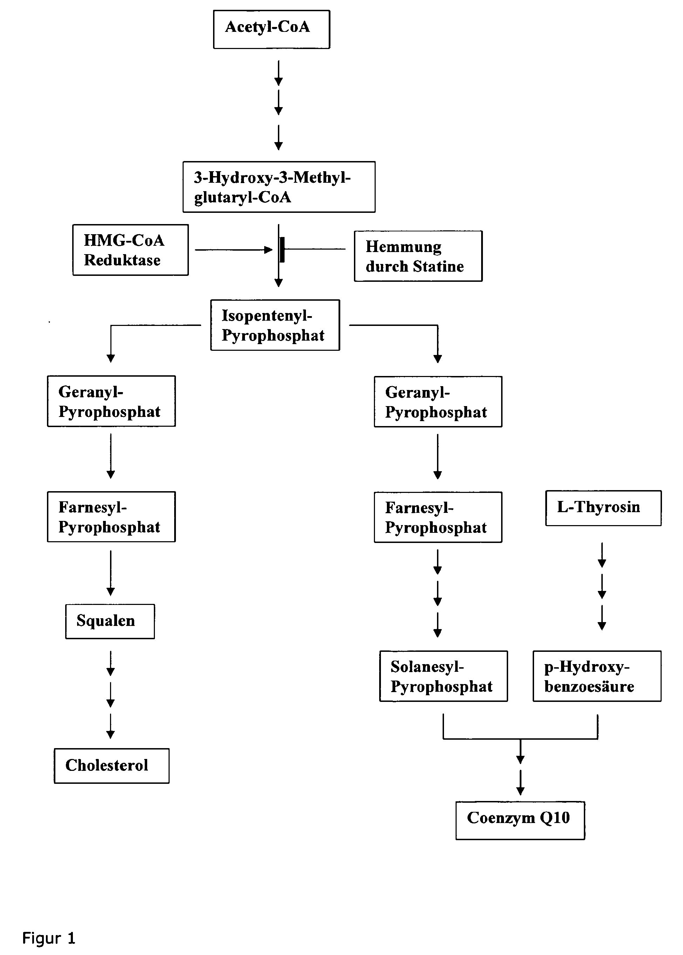

Die Zusammenhänge sind in der Figur 1 dargestellt.

In der kanadischen Ausgabe des NEJM aus dem Jahre 2004 wurde in einer kommerziellen Anzeige bezüglich der Statine Atrovastatin (Lipitor^{®}, Sortis^{®}) und Rosuvastatin (Crestor^{®}) darauf hingewiesen, dass unter Gabe dieser Arzneimittel signifikante Abfälle der Serum Coezym Q10-Konzentration zu beobachten waren.

Die langfristige klinische Signifikanz der Langzeitsuppression der Q10 Synthese durch Statine sei noch nicht bekannt, aber ein Abfall der myokardialen Q10 Konzentration könne bei Patienten mit grenzwertigen kongestiven Herzversagen zur Verschlechterung der Herzfunktion führen.

Im Rahmen der vorliegenden Erfindung werden die Begriffe Coenzym Q10, Q10 und Ubichinon-10 synonym verwendet.

Tatsächlich ist diese offensichtlich Nebenwirkung seit langem bekannt. 1990 ließ sich die Fa Merck die Gabe von Coenzym Q10 in Kombination mit Statinen zur Behandlung von "HMG-CoA-Reduktasehemmern verursachter Myopathie", patentrechtlich schützen. (US Patentschrift 4,929,437). Erfinder ist Micheal S. Brown, der zusammen mit Josef L. Goldstein 1985 den Nobelpreis für Medizin erhielt, für ihre Forschungen bezüglich des LDL-Rezeptors.

Auch im Zusammenhang mit der Fibromyalgie ist Coenzym Q10 geeignet, die Symptomatik, wie auch die Kausalität dieser Erkrankung nachhaltig zu verbessern. Fibromyalgie ist eine sehr komplexe Erkrankung, bei der es durch vermehrte Radikalbildung unter anderem zu einer mitochondrialen Dysfunktion kommt. Oberstes Ziel muss es daher sein, die Radikalbildung zu reduzieren, sowie die Optimierung der mitochomdrialen Leistungsfähigkeit zu gewährleisten.

Sehr beachtlich ist in diesem Zusammenhang die Entdeckung, dass bei all diesen Erkrankungen, niedrige Coenzym Q10 Plasmaspiegel invers korrelieren mit der Dauer der Erkrankung.

Der Untergang des zellulären mitochondrialen Netzwerkes ist ein schleichender Prozess. Können anfangs intakte Mitochondrien den Ausfall einzelner Mitochondrien kompensieren tritt zwangsläufig der Punkt ein, in der die Zelle in ein energetisches "Loch" fällt und untergeht.

Nicht nur oben beschriebene Krankheiten, sondern auch der Alterungsprozess allgemein scheinen ihrem Ausgangspunkt in den Mitochondrien zu haben.

Das Energieniveau einer Zelle im Körper hängt wesentlich von der Q10 Konzentration in den mitochondrialen Membranen ab (Müller U., Kriegelstein J., Sauerstoffradikalfänger als Neuroprotektiva, Dt 18, (1994) S. 17).

Für die Bereitstellung von Energie hat Q10 eine Schlüsselposition. Keine andere Substanz kann sie ersetzen.

### Betrachtung: Energiegewinnung aus Glucose:

| | |
|---|---|
| Glycolyse: | 2 NADH + 2 ATP |
| Pyruvat, Oxidation zu AcetylCoA | 2 NADH |
| Citratcyclus | 6 NADH + 2 FADH₂ |
| | 10 NADH + 2 FADH₂ + 2 ATP |

Jedes NADH liefert 3 ATP. Jedes FADH₂ liefert 2 ATP. Ergibt in Summe 10 x3 +2x2 +2 = 36 ATP. NADH liefert also insgesamt 30 ATP. NADH muss allerdings zwingend seine energiereichen Elektronen an Coenzym Q10 abgeben. Dass bedeutet von insgesamt 36 ATP die ein Molekül Glucose liefern, müssen 30 ATP via NADH über Coenzym Q10 abreagieren (FADH₂ gibt seine Elektronen an die Cytochromoxidase ab, also vorbei an Coenzym Q10). Kommt es zu einem chronischen Q10 Mangel können energiereiche Elektronen von NADH kommend direkt auf Sauerstoff übertragen werden. Als Folge können wie oben beschrieben Radikale entstehen.

### Mitochondriale Folgen:

Mitochondriale Enzymkomplexe der Atmungskette werden blockiert. Dadurch entsteht ein ATP Mangel. Die Fettverbrennung wird dadurch behindert, da diese ausschließlich in den Mitochondrien erfolgt. Gleichzeitig wird zur Deckung des ATP Bedarfs die Glykolyse aktiviert, die aber nur etwa 1/16 der ATP Ausbeute liefert, im Vergleich zur ATP Ausbeute in der mitochondrialen Atmungkette. Energiebedürftige Organe sind besonders betroffen wie etwa Herz und Muskeln, aber auch das zentrale Nervensystem. Es resultiert eine reichliche Kohlenhydratzufuhr, die das radikalische Geschehen weiter unterstützt.

Leichte Mitochondriopathien erzeugen Adipositas, Hypertonie und den Typ-II-Diabetes, auch bekannt als metabolisches Syndrom. In Folge steigen ox-LDL-Cholesterin, nicht selten der Liporotein-a Spiegel, als auch das C-reaktive Protein.

Dass mit zunehmendem Alter des Menschen die körpereigene Q-10 Produktion tatsächlich nachlässt konnte Kalén zeigen (Kalen A: in Bliznakov EG, Hunt GL: Die Entdeckung: Energie-Vitamin Q10. Lebensbaum Verlag.)

Um mitochondriale Funktionalität wieder herzustellen bedarf es vor allem Coenzym Q10. Prinzipiell wird Q10, als Vitaminoid, in der Leber bereitgestellt. Aus der Aminosäure Tyrosin wird der Chinonring gebildet und parallel zur Cholesterinsynthese wird, via Mevalonat, die Isoprenylseitenkette geliefert, die im Rahmen einer Alkylierung in ortho-Position neben der aromatischen Hydroxyfunktion angebracht wird.

Die Leber verliert allerdings im Alter an Leistungsfähigkeit. Es wird zwar immer noch der Q10 Gehalt der Nahrung genutzt, minderwertige Ubichinon-Verbindungen wie Coenzym Q7-Q9 können aber nicht mehr ausreichend zu Coenzym Q10 aufgebaut werden.

Die mitochondriale DNA Mutation und fortschreitende Verschlechterung des zellulären bioenergetischen Status sind Mechanismen des Altern. Die Zelle altert mittels ihrer Mitochondrien.

Coenzym Q10 ist eine der entscheidenden Schlüsselsubstanzen in der mitochondrialen Atmungskette, sie transportiert Elektronen vom Komplex I + II zum Komplex III. Nützlich wäre deshalb die Zufuhr geeigneter Mengen an Coenzym Q10.

Herkömmliche pulverförmige oder in Öl gelöste Formulierungen werden aber nur schlecht resorbiert.

Solubilisierte Formulierungen enthalten entweder Alkohol oder haben wie im Fall DE 102 55 195 A1 relativ große Mengen an lipophilen Substanzen. Hier wird ein Lecithin Fett Verhältnis von 1:1 bis 1:12 beschrieben, bei einer Konzentration der Mischung Lecithin/Lipid in der Polyol-Komponente von 10 Gew.-% bis 90.-Gew%. Durch den relativ hohen Lipid-Anteil ist grundsätzlich ein Aroma bei trinkbaren Formulierungen erforderlich. Gleichzeitig resultieren sehr häufig gastrointestinale Unverträglichkeiten, wie Magendrücken und unangenehmes Aufstoßen. Folglich ist die Akzeptanz dieser Formulierungen nur gering.

WO 2008/000534 A1 offenbart Solubilisationsformulierungen. Es werden aber keine Hinweise auf ein spezielles Gewichtsverhältnis von Phospholipid zu lipophilen Substanzen gegeben. Lediglich zusammenhanglos werden zahlreiche Bestandteile angegeben ohne das Ziel physiologisch gut verträglicher und geschmacklich hervorragender Emulsionen zu offenbaren, was überraschend in einem eng begrenzten Phospholipid/Lipid-Verhältnis gefunden wurde. In den Ausführungsbeispielen werden zudem Lecithin/Lipophile Substanz-Verhältnisse von 1:4 offenbart. In den Ausführungsbeispielen 1 und 2 werden beispielsweise 20% Vitamin E bzw. 20% Coenzym Q10 offenbart. Keines der Ausführungsbeispiele offenbart ein Lecithin/lipophile Substanz-Verhältnis zwischen 1:0,99 und 1:0,1. Zudem offenbart kein Beispiel den Einsatz von Citronensäure bzw. Citraten. Lediglich zur Sprühtrocknung werden diese erwähnt. Keines der Ausführungsbeispiele legt die in dieser Anmeldung offenbarte Zusammensetzung als trinkbare Zusammensetzung nahe.

WO 2006/042574/A2 offenbart Solubilisationsformulierungen, bestehend aus einer wässrigen Lösung von Alkali und/oder Erdalkalisalzen der Ascorbinsäure sowie einem Emulgator mit einem HLB-Wert von 9 bis 18, insbesondere Polysorbate für Lebensmittel, Kosmetika und dergleichen. Die hier eingesetzten Emulgatoren sind bitter und bedürfen zur oralen Applikation grundsätzlich eines Aromas. Zudem haben synthetische Emulgatoren oft negative Auswirkungen auf die Gesundheit, wie etwa ein gesteigertes allergenes Potential.

Die im Stand der Technik aufgezeigten solubilisierten Coenzym Q10 Formulierungen enthalten entweder Alkohol, zu hohe lipophile Anteile oder synthetisch bitter schmeckende Emulgatoren.

Aufgabe der vorliegenden Erfindung ist es, langzeitstabile Emulsionen bereitzustellen, die geeignet sind hydrophile, als auch lipophile Substanzen, wirkungsvoll zu solubilisieren, wobei gleichzeitig eine hohe physiologische Verträglichkeit, sowie ein angenehmer Geschmack, ohne Zugabe von Aromen, Konservierungsmitteln und Emulgatoren möglich ist.

Eine weitere Aufgabe bestand darin, ein verbessertes pharmazeutisches Präparat zur Verfügung zu stellen, das zur Behandlung oder Prophylaxe der mitochondrialen Dysfunktion eingesetzt werden kann.

Überraschend wurde nun gefunden, dass die erfindungsgemäßen speziellen flüssigen, wässrigen Emulsionen, die im Stand der Technik genannten Probleme lösen können.

Gegenstand der vorliegenden Erfindung in einer ersten Ausführungsform ist eine flüssige wässrige Emulsion umfassend
a) ein oder mehrere Phospholipid(e),
b) ein oder mehrere Polyole ausgewählt aus der Gruppe bestehend aus Glycerin und/oder Saccharid(en), und
c) lipophile Komponenten umfassend mittelkettige Triglyceride und Ubichinon-10,
wobei das Gewichtsverhältnis von Phospholipid zum Gesamtgewicht der lipophilen Komponenten 1:0,1 bis 1:0,98 beträgt.

Die erfindungsgemäßen Emulsionen enthalten als wesentlichen Bestandteil ein oder mehrere Phospholipid(e).

Phospholipide sind Phosphorsäuredi- oder monoester, die wegen ihrer fettähnlichen Löslichkeitseigenschaften auf Grund der lipophilen und hydrophilen Komponenten zu den Lipiden gerechnet werden und im Organismus als Membranlipide am Aufbau von Schichtstrukturen der Membran beteiligt sind. Besonders reichlich sind Phospholipide vorhanden in der Hirnsubstanz und im Myelin. Eine besondere Gruppe der Phospholipide, die sich vom Sphingosin ableitet, sind die sogenannten Phosphosphingolipide. Die erfindungsgemäßen Zusammensetzungen enthalten jedoch bevorzugt Phospholipide, die Derivate des Glycerins darstellen und auch als Phosphoglyceride oder Phosphatide bezeichnet werden.

In der Lipid-Nomenklatur hat sich für asymmetrische Glycerin-Derivate die sogenannten stereospezifische Numerierung (Abk.: sn-, nach IUPAC/IUBMB-Regel Lip-1.13) als nützlich erwiesen, bei der die (freie oder substituierte) Hydroxy-Gruppe in Position 2 des Glycerin-Teils nach links weisend und das geminale Wasserstoff-Atom nach rechts gezeichnet und als über die Papierebene ragend gedacht werden, woraufhin das Kohlenstoff-Atom 1 des Glycerin-Teils nach oben, Kohlenstoff-Atom 3 jedoch nach unten gerichtet sind.

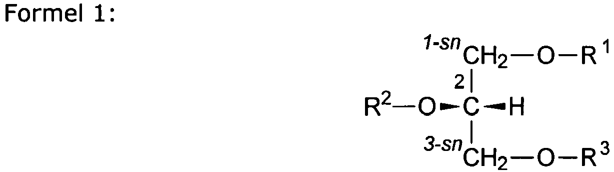

Phosphatidsäuren sind Glycerin-Derivate, die in 1-sn- und 2-Stellung mit Fettsäuren (1-sn-Position: meist gesättigt, 2-Position: meist ein- od. mehrfach ungesättigt), an Atom 3-sn dagegen mit Phosphorsäure verestert sind (Formel 1: R1, R2 = Acyl, R3 = Phosphoryl). Die Phosphatidsäuren selbst haben wahrscheinlich regulatorische Funktionen für Cytoskelett, Membrantransport und Sekretion, werden durch Phospholipase D aus Phosphatiden freigesetzt und nur in kleinen Konzentration im Gewebe gefunden; ihr Phosphat-Rest ist meist verestert mit Aminoalkoholen wie Cholin (Lecithin = 3-sn-Phosphatidylcholin) oder 2-Aminoethanol (Ethanolamin) bzw. L-Serin (Kephalin = 3-sn-Phosphatidylethanolamin bzw. -L-serin), mit myo-Inosit zu den in Geweben häufigen Phosphoinositiden [1-(3-sn-Phosphatidyl)-D-myo-inositen], mit Glycerin zu den im Fruchtwasser nachgewiesenen Phosphatidylglycerinen, die auch beim Protein-Export aus Gram-neg. Bakterien eine Rolle spielen sollen. Cardiolipine (1,3-Bisphosphatidylglycerine) sind aus Mitochondrien-Membranen des Herzmuskels isolierte Phospholipide aus zwei über Glycerin verknüpften Phosphatidsäuren. Lysophospholipide erhält man, wenn aus Phospholipiden ein Acyl-(Fettsäure-)Rest durch Phospholipase A abgespalten wird; Beispiel: Lysolecithine. Phosphatasen u. Phosphodiesterasen wie die Phospholipasen C und D spalten Phospholipide demgegenüber an den Phosphat-Bindungen. Zu den Phospholipiden rechnet man ferner die Plasmalogene, in denen statt einer Fettsäure in 1-Stellung ein Aldehyd (in Form eines Enolethers) gebunden ist; die den Phosphatidylcholinen entsprechenden O-1-sn-Alkenyl-Verb. z. B. heißen Phosphatidalcholine. Vom Namen Plasmalogen leitet sich die Sammelbezeichnung Plasmensäuren her für Phospholipide mit einer Enolether-Gruppierung in 1-, einer (ungesättigten) Acyl-Gruppe in 2- und einer Phosphorsäure-Gruppe in 3-Stellung; die entsprechenden 1-Alkylether-Derivate heißen Plasmansäuren und desacylierte Derivate beispielsweise Lysoplasmenylserin. Ein weiteres Phospholipid ist der Thrombocyten-aktivierende Faktor (platelet-activating factor, PAF, siehe Formel 1 mit R1 = Octadecyl, R2 = Acetyl und R3 = Phosphorylcholin). In Ciliaten-Membranen wurden Phosphonolipide entdeckt, deren Funktion möglicherweise darin besteht, ein Überleben in Anwesenheit von - selbst sezernierten - Phospholipasen zu ermöglichen.

Phosphatidyl-L-serine und Phosphatidylinosite entstehen durch enzymatische Reaktion von L-Serin bzw. myo-Inosit mit 3-CDP-1,2-Diacyl-sn-glycerinen unter Freisetzung von Cytidin-5'-monophosphat. Phosphatidylethanolamine bilden sich bei Decarboxylierung von Phosphatidyl-L-serinen, Phosphatidylcholine wiederum durch schrittweise Methylierung der Ethanolamin-Derivate. Andererseits können freigesetztes Cholin und Ethanolamin mit Hilfe von Cytidin-5'-triphosphat aktiviert werden zu den CDP-Derivaten und dann mit 1,2-Diacyl-sn-glycerinen unter Abspaltung von CMP zu den korrespondierenden Phosphoglyceriden reagieren.

Besonders bevorzugt enthalten die erfindungsgemäßen Emulsionen Lecithin als Phospholipid. Lecithin ist die Gruppenbezeichnung für diejenigen Glycerophospholipide, die sich aus Fettsäuren, Glycerin und Phosphorsäure und Cholin durch Veresterung bilden. Heute hat sich die Bezeichnung Phosphatidylcholin weitgehend durchgesetzt.

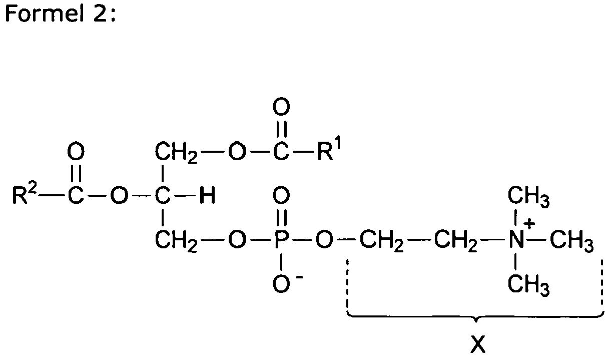

Allg. Struktur der Lecithine; R1, R2: typischerweise unverzweigte aliphatische Reste mit 15 oder 17 Kohlenstoff-Atomen und bis zu 4 cis-Doppelbindungen.

Die in der Natur vorkommenden Lecithine sind, wie die eng verwandten Kephaline, Derivate der 1,2-Diacyl-sn-glycerin-3-phosphorsäuren (Phosphatidsäuren; X = H; R2-CO-O- in Fischer-Projektion nach links weisend) besitzen also α-Konfiguration. Beim β-Lecithin ist der mit Cholin veresterte Phosphorsäure-Rest an die mittelständige Hydroxy-Gruppe eines Glycerids gebunden. Aus der Verschiedenheit der Fettsäure-Reste R1 und R2 ergibt sich eine große Zahl verschiedener Lecithine. Bei Extraktionen aus biologischen Material erhält man immer Gemische. So enthält eine Lecithin-Fraktion aus Sojabohnen beispielsweise Palmitinsäure, Stearinsäure, Palmitoleinsäure, Oleinsäure, Linolsäure und Linolensäure. Normalerweise ist die gesättigte Fettsäure mit der primären, die ungesättigte mit der sekundären Hydroxy-Gruppe des Glycerins verestert.

In einer bevorzugten Ausführungsform der vorliegenden Erfindung enthalten die erfindungsgemäßen Emulsionen Phospholipid, das aus Soja und/oder Raps und/oder Ei gewonnen wurde.

Weiter bevorzugt ist Phospholipid, das aus Raps, Fisch oder Milch gewonnen wurde.

Weiter bevorzugt enthalten die erfindungsgemäßen Emulsionen hydrierte und/oder hydrolysierte und/oder hydroxylierte Phospholipide.

Speziell bevorzugt sind erfindungsgemäße Emulsionen, die Lecithin, insbesondere Soja und/oder Eilecithin enthalten.

Das/die Phospholipid(e) liegen in den erfindungsgemäßen Emulsionen vorzugsweise in einer Menge von 0,4 bis 50 Gew.-%, weiter bevorzugt von 0,7 bis 40 Gew.-%, besonders bevorzugt von 2 bis 20 Gew.-% und insbesondere von 2,5 bis 15 Gew.-%, speziell von 4 bis 10 Gew.-% und äußerst bevorzugt von 4 bis 6 Gew.-%, jeweils bezogen auf die gesamte Emulsion, vor.

In einer besonders bevorzugten Ausführungsform enthalten die Phospholipide mindestens 40 Gew.-% Phosphatidylcholin, bezogen auf das Gesamtgewicht der Phospholipide.

Ein weiterer wesentlicher Bestandteil der erfindungsgemäßen flüssigen wässrigen Emulsion ist ein Polyol, welches ausgewählt sein kann aus der Gruppe bestehend aus Glycerin und/oder Saccharid(en).

Die Saccharide können dabei bevorzugt aus der Gruppe der Monosaccharide, Disaccharide und/oder Oligosaccharide ausgewählt sein. Als besonders vorteilhaft hinsichtlich des Geschmacks und der Verträglichkeit der Emulsion haben sich Fructose, Mannitol, Maltodextrin, Glucose und/oder dessen physiologisch akzeptable Derivate herausgestellt. Besonders bevorzugt ist Fructose und/oder Glucose.

In einer bevorzugten Ausführungsform weist die erfindungsgemäße flüssige wässrige Emulsion 30 bis 70 Gew.-% Polyol(e), weiter bevorzugt 42 bis 58 Gew.-% und insbesondere 45 bis 55 Gew.-% Polyol(e), jeweils bezogen auf das Gesamtgewicht der Emulsion, auf.

Ein weiterer wesentlicher Bestandteil der erfindungsgemäßen wässrigen Emulsion sind lipophile Komponenten, die zumindest mittelkettige Triglyceride und Ubichinon-10 umfassen.

Das Gewichtsverhältnis von Phospholipid zum Gesamtgewicht der lipophilen Komponenten beträgt 1:0,1 bis 1:0,98, vorzugsweise 1:0,02 bis 1:0,9, insbesondere 1:0,3 bis 1:0,8 und im Speziellen 1:0,4 bis 1:0,75.

Ein wesentlicher Bestandteil der lipophilen Komponenten sind die mittelkettigen Triglyceride sowie das Ubichinon-10.

Mittelkettige Triglyeride (MCT) sind Fette mit einer speziellen Molekülstruktur und Löslichkeit. MCT werden vom Körper nicht in das Fettgewebe eingelagert, vermindern den Cholesterinspiegel und versorgen den Körper rasch und über längere Zeit mit hochwertiger Energie und werden auch lange vor den Proteinen verbrannt. Mittelkettige Triglyceride sind Esterverbindungen aus mittelkettigen Fettsäuren und Glycerin. Die mittelkettigen Fettsäuren bestehen aus C₆-C₁₂ Fettsäuren. Insbesondere bevorzugt sind mittelkettige Triglyceride, die durch Veresterung von Glycerin mit Capronsäure, Caprylsäure, Caprinsäure und/oder Laurinsäure erhalten werden können. Der Anteil mittelkettiger Triglyceride beträgt vorzugsweise 0,5 bis 10 Gew.-%, weiter bevorzugt 1 bis 8 Gew.-%, insbesondere 2 bis 5 Gew.-%, jeweils bezogen auf das Gesamtgewicht der Emulsion.

Der Anteil an Ubichinon-10 (Coenzym Q10) beträgt vorzugsweise 0,2 bis 10 Gew.-%, weiter bevorzugt, 0,3 bis 5 Gew.-% und insbesondere 0,5 bis 2 Gew.-%, jeweils bezogen auf das Gesamtgewicht der erfindungsgemäßen Emulsion.

Die erfindungsgemäße Emulsion kann darüber hinaus weitere lipophile Komponenten aufweisen. In einer bevorzugten Ausführungsform weisen die erfindungsgemäßen Emulsionen zusätzliche lipophile Komponenten auf, die ausgewählt sind aus der Gruppe bestehend aus Fettsäuretriglyceriden, die keine mittelkettigen Triglyceride sind, Vitamin E, Vitamin D, Vitamin K, Vitamin A, Liponsäure, Karotinoide, Tocopherole, Tocotrienole und beliebige Mischungen hiervon.

Als lipophile Komponenten kommen weiterhin insbesondere Vitamine und Carotinoide in Betracht. In einer bevorzugten Ausführungsform enthalten die erfindungsgemäßen Emulsionen ein oder mehrere lipophile Stoff(e), ausgewählt aus der Gruppe bestehend aus Triglyceride enthaltend C₁₀-C₂₂ Fettsäuren, Isoprenoide, Terpene, Citral, Menthol, Kampfer, Vitamin A, Vitamin K, E-Vitamine sowie deren physiologisch verträgliche Derivate, z.B. Tocopherolacetat, Tocotrienole, Karotinoide, beta-Karotin, Lutein, Zeaxanthin, Lycopin und deren physiologisch verträgliche Derivate.

Die lipophilen Komponenten weisen vorzugsweise einen HLB-Wert < 10 auf. Der HLB-Wert wird nach der Methode von Griffin bestimmt (Griffin, W.C.: Classification of Surface Active Agents by HLB, J. soc. Cosmet. Chem. 1, 1949).

In einer weiteren bevorzugten Ausführungsform beträgt das Gesamtgewicht von Phospholipid(en) und lipophilen Komponenten 5 bis 30 Gew.-%, weiter bevorzugt 6 bis 20 Gew.-% und insbesondere 7 bis 15 Gew.-%, jeweils bezogen auf das Gesamtgewicht der Emulsion.

Hervorragende Eigenschaften können insbesondere mit solchen erfindungsgemäßen Emulsionen erzielt werden, bei denen das Gewichtsverhältnis von Polyol zu Phospholipid 25:1 bis 3:1, vorzugsweise 18:1 bis 5:1 und insbesondere 12:1 bis 8:1 ist.

In einer weiter bevorzugten Ausführungsform enthalten die erfindungsgemäßen Emulsionen zusätzlich Zitronensäure und/oder Alkali- und/oder Erdalkalicitrate. Besonders bevorzugt eingesetzt wird Magnesiumcitrat.

Die Zitronensäure und/oder die Citrate können in einer bevorzugten Ausführungsform in einer Menge von 0,1 bis 5 Gew.-%, vorzugsweise 2 bis 4 Gew.-% vorliegen.

Darüber hinaus wurde überraschend gefunden, dass die erfindungsgemäßen Emulsionen durch den Zusatz von Ascorbinsäure weiter stabilisiert werden können. In einer bevorzugten Ausführungsform enthalten die erfindungsgemäßen Emulsionen daher Ascorbinsäure.

Vorzugsweise liegt die Menge an Ascorbinsäure in einem Bereich von 0,1 bis 5 Gew.-%, vorzugsweise 0,5 bis 4 Gew.-%, insbesondere 1 bis 3 Gew.-%.

Der Wassergehalt der flüssigen wässrigen Emulsion liegt in einer bevorzugten Ausführungsform im Bereich von 5 bis 65 Gew.-%, vorzugsweise 9 bis 55 Gew.-% und insbesondere 25 bis 45 Gew.-%, jeweils bezogen auf die gesamte wässrige Emulsion vor.

Die erfindungsgemäßen wässrigen flüssigen Emulsionen liegen bevorzugt micellar vor. Insbesondere in der micellaren Form sind die erfindungsgemäßen Emulsionen geeignet, die lipophilen Komponenten zu solubilisieren. Die Ausbildung von Micellen macht es auch möglich, konzentrierte flüssige Formulierungen herzustellen, die beispielsweise ein Wassergehalt unterhalb von 40 Gew.-% aufweisen. In einer bevorzugten Ausführungsform liegen die flüssigen wässrigen Emulsionen als micellares Konzentrat vor. Die Micellen in den erfindungsgemäßen Emulsionen weisen bevorzugt einen mittleren Durchmesser von 10 bis 150 nm, vorzugsweise 25 bis 90 nm, insbesondere 55 bis 75 nm auf. Die flüssigen wässrigen Emulsionen gemäß der vorliegenden Erfindung sind bevorzugt transparent.

Der mittlere Durchmesser der Micellen kann mit dem Fachmann geläufigen Verfahren bestimmt werden, beispielsweise einem Coulter Counter N24.

Die erfindungsgemäßen Emulsionen weisen eine überraschend gute Lagerstabilität bei 25°C auf und eigenen sich insbesondere für die orale Anwendung. Die erfindungsgemäßen Emulsionen können länger als 1 Jahr stabil gelagert werden.

In einer weiteren bevorzugten Ausführungsform sind die erfindungsgemäßen Emulsionen im Wesentlichen frei von Aromen und/oder künstlichen Emulgatoren. Im Wesentlichen frei im Rahmen der vorliegenden Erfindung bedeutet, dass die entsprechenden Komponenten in einer Menge unterhalb von 1 Gew.-%, vorzugsweise unterhalb von 0,1 Gew.-%, insbesondere weniger als 0,01 Gew.-%, im Speziellen weniger als 0,001 Gew.-%, beispielsweise unterhalb von 0,00001 Gew.-%, jeweils bezogen auf das Gesamtgewicht der Emulsion, aufweisen.

In einer besonderen Ausführungsform der vorliegenden Erfindung basiert die erfindungsgemäße Emulsion im Wesentlichen auf orthomolekularen Bestandteilen. Dies ist insbesondere im Bereich der orthomolekularen Medizin von Bedeutung, da nicht nur die eingesetzten Nährstoffe, sondern das gesamte Produkt hervorragend verträglich sein soll. In einer bevorzugten Ausführungsform weist die erfindungsgemäße Emulsion daher keine Substanz auf, die dem Körper nicht von Natur aus bekannt ist.

In einer weiteren bevorzugten Ausführungsform enthalten die erfindungsgemäßen Emulsionen zusätzlich Vitamine, Aminosäure, Mineralien bzw. Spurenelemente sowie sekundäre Pflanzeninhaltsstoffe.

Bevorzugte Vitamine sind:
- 1. Vitamine der B-Reihe: Thiamin (B₁), Riboflavin (B₂), Nicotinamid, Nicotinsäure (Niacin B₃), Panthothensäure (B₅), Pyridoxal (B₆,) Cobalamin (B₁₂), Folsäue, Biotin (Vitamin H)
- 2. Kalium-, Calcium-, Natrium-, Magnesiumascorbat sowie Ascorbylpalmitat.
- 3. Liponsäure und/oder deren physiologisch akzeptablen Derivate, Kreatin, L-Carnitin und/oder deren physiologisch akzeptablen Derivate.
- 4. Aminosäure und Peptide: Glycin, Alanin, Leucin, Isoleucin, Valin, Lysin, Methionin, Phenylalanin, Threonin, Arginin, Cystein, Serin, Glutamat, Glutaminsäure, Aspartat, Asparaginsäure, Prolin, Taurin, Glutathion und Carnosin.

Weiterhin bevorzugt enthält die erfindungsgemäße Emulsion zusätzlich Mineralstoffe und/oder Spurenelemente: Geeignete Mineralstoffe und Spurenelemente sind Kalium, Calcium, Magnesium, Natrium, Selen, Zink, Eisen, Jod, Fluor, Chrom, Kupfer, Mangan, Molybdän, Vanadium, Bor in Form ihrer physiologisch verträglich Derivate.

Weiterhin können die erfindungsgemäßen Formulierungen sekundäre Pflanzeninhaltsstoffe aus der Gruppe der Stilbene, Flavonoide, in Form von Resveratrol, OPC, Rutin, Quercetin, Naringin oder die pflanzlichen Extrakte wie z.B. Heidelbeerextrakt oder Traubenkernextrakt enthalten.

Weiterhin können die erfindungsgemäßen Formulierungen Coffein und/oder Coffein-haltige Pflanzenextrakte wie Guaranaextrakt enthalten.

Die erfindungsgemäßen Formulierungen können auch getrocknet werden.

Ein weiterer Gegenstand der vorliegenden Erfindung ist daher ein Feststoff, erhältlich aus einer erfindungsgemäßen Emulsion durch Trocknung, vorzugsweise Sprühtrocknung oder Gefriertrocknung. Es hat sich dabei herausgestellt, dass die getrockneten Feststoffe beim Auflösen des Feststoffs in Wasser die ursprüngliche Emulsion zurückgewonnen werden kann.

Ein weiterer Gegenstand der vorliegenden Erfindung ist ein Kit of Parts, umfassend die getrockneten Emulsionen sowie die genannten Vitamine, Aminosäure, Mineralien bzw. Spurenelemente sowie sekundäre Pflanzeninhaltsstoffe, wobei wenigstens eine der Komponenten räumlich getrennt von den anderen vorliegt. Das Kit of Parts kann dabei ein Behältnis sein, in dem sich zwei oder mehrere Kompartimente befinden, in den die Komponenten enthalten sind. In dem erfindungsgemäßen Kit können die einzelnen Komponenten unabhängig voneinander in Form eines Pulvers, Granulat, getrockneten Pulvers, oder daraus gewonnenen Tabletten, Dragees, Kapseln vorliegen. In einer bevorzugten Ausführungsform weist das erfindungsgemäße Kit mindestens ein Kompartiment auf und einen zusätzlichen Behälter, wobei in wenigstens einem der Kompartimente und/oder in dem Behälter zwei der Komponenten ausgewählt aus den Komponenten getrocknete Emulsion bis Vitamine, Aminosäure, Mineralien bzw. Spurenelemente sowie pflanzliche Inhaltsstoffe bzw. Pflanzenextrakte enthalten sind.

Ein weiterer Gegenstand der Erfindung ist ein Getränk, das die erfindungsgemäße Emulsion oder den erfindungsgemäßen Feststoff z.B. ein aus der Emulsion hergestelltes Pulver, enthält.

Ein weiterer Gegenstand der vorliegenden Erfindung sind Lutschbonbons oder Kaumassen, die die erfindungsgemäßen Emulsionen oder den erfindungsgemäßen Feststoff enthalten.

Ein weiterer Gegenstand der vorliegenden Erfindung ist die Verwendung der flüssigen wässrigen Emulsion als Getränk, insbesondere als Diätetikum.

Ein weiterer Gegenstand der vorliegenden Erfindung ist eine pharmazeutische Zubereitung, umfassend die erfindungsgemäße flüssige wässrige Emulsion. Es hat sich überraschend gezeigt, dass die erfindungsgemäße pharmazeutische Zubereitung zur Prophylaxe und Therapie mitochondrialer Dysfunktionen eingesetzt werden kann.

Ein weiterer Gegenstand der vorliegenden Erfindung ist daher die erfindungsgemäße pharmazeutische Zubereitung zur Verwendung in der Therapie und Prophylaxe von mitochondrialen Dysfunktionen.

Darüber hinaus wurde festgestellt, dass die mit mitochondrialen Fehlfunktionen im Zusammenhang stehenden Erkrankungen mit den erfindungsgemäßen pharmazeutischen Zubereitungen ebenfalls behandelt werden können. Ein weiterer Gegenstand der vorliegenden Erfindung ist daher die erfindungsgemäße pharmazeutische Zubereitung zur Behandlung oder Prophylaxe von Erkrankungen, die durch mitochondriale Dysfunktionen verursacht werden, insbesondere ausgewählt aus der Gruppe Typ-II Diabetes, Arteriosklerose, Kardiomyophatie, Fibromyalgie, Alzheimersche Krankheit, Parkinsonsche Krankheit, Adipositas und Hypertonie.

### Ausführungsbeispiel 1: Erfindungsgemäße Emulsion

| Rohstoff | Menge in Gewichtsprozent |
|---|---|
| Fructose | 50,0 |
| L-Carnitin | 5,0 |
| Phosphatidylcholin | 4,5 |
| Mittelkettige Triglyceride | 3,0 |
| Coenzym Q10 | 1,0 |
| Ascorbinsäure | 2,0 |
| Magnesiumcitrat | 3,0 |
| Wasser | ad 100 |

Wasser, Fructose, L-Carnitin Ascorbinsäure. Magnesiumcitrat, mittelkettige Triglyceride und Coenzym Q10 werden im Hochdruckhomogenisator bei 400 bar homogenisiert.
Die Emulsion kann mittels Gefriertrocknung getrocknet werden. Es werden rieselfähige Pulver erhalten.

### Ausführungsbeispiel 2: Erfindungsgemäße Emulsion

| Rohstoff | Menge in Gewichtsprozent |
|---|---|
| Fructose | 50,0 |
| L-Carnitin | 4,5 |
| Phosphatidylcholin | 4,5 |
| Mittelkettige Triglyceride | 3,0 |
| Coenzym Q10 | 1,0 |
| Alpha-Tocopherol | 0,5 |
| Ascorbinsäure | 2,0 |
| Magnesiumcitrat | 3,0 |
| Wasser | ad 100 |

Wasser, Fructose, L-Carnitin Ascorbinsäure. Magnesiumcitrat, mittelkettige Triglyceride, Alpha-Tocopherol und Coenzym Q10 werden im Hochdruckhomogenisator bei 400 bar homogenisiert.

**Studie II:** 12 Personen im Alter von 29 bis 64 nahmen an einer Versuchsreihe teil. 6 Personen erhielten 20 g der Emulsion (Beispiel 2), 6 Personen erhielten in pulverform verkapselt 3 Kapseln enthaltend:
1. Kapsel 1000 mg L-Carnitin,
2. Kapsel 200 mg Coenzym Q10, 400 mg Ascorbinsäure und
3. Kapsel 600 mg Magnsesiumcitrat.
Der Coenzym Q10 Spiegel im Blut stieg mit der eingesetzten Emulsion deutlich stärker an. Nach 4 Stunden konnten Blutplasmakonzentrationen zwischen 6 und 9,5 µg/ml nachgewiesen werden, im Gegensatz zur Pulverformulierung, bei der lediglich Blutplasmakonzentrationen zwischen 2,3 und 3,1 µg/ml nachgewiesen wurden.

**Studie II:** 14 Personen im Alter von 27 bis 59 nahmen an einer Studie teil. 7 Personen erhielten 20 g der Emulsion (Beispiel 2), 7 Personen 20 g der folgenden Emulsion (Vergleichsemulsion). Getestet wurde auf evtl. auftretende gastointestinale Störungen

### Vergleichsemulsion :

| Komponente | Menge in Gewichtsprozent |
|---|---|
| Phosphatidylcholin | 5,0 |
| Mittelkettige Triglyceride | 5,0 |
| Coenzym Q10 | 1,0 |
| Alpha-Tocopherol | 0,5 |
| Ascorbinsäure | 2,0 |
| Magnesiumcitrat | 3,0 |
| Wasser | ad 100 |

### Ergebnis:

| Ausführungsbeispiel 2 | Vergleichsemulsion |
|---|---|
| 1 gut verträglich | gut verträglich |
| 2 gut verträglich | Aufstoßen und leichtes Magendrücken |
| 3 gut verträglich | gut verträglich |
| 4 gut verträglich | leichtes Aufstoßen |
| 5 gut verträglich | gut verträglich |
| 6 gut verträglich | Aufstoßen |
| 7 gut verträglich | leichtes Aufstoßen |

### Ausführungsbeispiel 3:

### Zusammensetzung der Emulsion:

| Komponente | Menge in Gramm [g] |
|---|---|
| Wasser | 330 |
| Fructose | 508 |
| L-Carnitin | 60 |
| Lecithin | 51 |
| MCT | 35 |
| Ubiquinon | 10 |
| Vitamin E | 5 |
| Vitamin C | 1 |

Verhältnis Lecithin zu allen lipophilen Komponenten 1:0,98

Die Emulsion gemäß Beispiel 3 wurde im Hochdruckhomogenisator bei 400 bar homogenisiert. Die Emulsion wurde 5 Probanden über einen Zeitraum von 16 Tagen zugeführt. Die Dosierung lag bei einer täglichen Applikation von 20 ml oral.

Das Blut des Probanden wurde zu Beginn des Versuchs (t1), also vor dem Applikationszeitraum und am 16. Tag der Applikation vermessen. Gemessen wurde NaF-Blut und es wurde der Lactat- und der Pyruvat-Gehalt bestimmt (Tabelle A)

**Tabelle A**

| Proband | Alter (Jahre) | Lactat (t1)¹⁾ | Pyruvat (t₁)²⁾ | L/P (t1)³⁾ | Lactat (t2)₄₎ | Pyruvat (t2)⁵⁾ | L/P (t2)⁶⁾ | Diff. Quotient⁷⁾ | Einnahmedauer (Tage) |
|---|---|---|---|---|---|---|---|---|---|
| 1 | 52,00 | 0,95 | 51,60 | 18,41 | 1,36 | 141,20 | 8,92 | -9,49 | 16 |
| 2 | 40,00 | 1,03 | 68,80 | 14,97 | 1,06 | 93,20 | 11,37 | -3,60 | 16 |
| 3 | 60,00 | 1,51 | 58,80 | 25,68 | 1,54 | 84,20 | 18,29 | -7,39 | 16 |
| 4 | 41,00 | 1,01 | 69,60 | 14,51 | 1,03 | 93,20 | 11.05 | -3,46 | 16 |
| 5 | 75,00 | 0,96 | 49,60 | 19,35 | 1,19 | 112,10 | 10,62 | -8,74 | 16 |
| Statistik Mittelwert: | 53,60 | 1,09 | 59,68 | 18,59 | 1,22 | 104,78 | 12,05 | -6,53 | 16 |
| Standardabweichung | 14,54 | 0,24 | 9,34 | 4,49 | 0,20 | 22,76 | 3,61 | 2,85 | |

| | | | | | | | | | |
|---|---|---|---|---|---|---|---|---|---|
| ¹⁾ Lactat (mmol/l) zum Zeitpunkt t1 = vor der Behandlung ²⁾ Pyruvat (mmol/l) zum Zeitpunkt t1 = vor der Behandlung ³⁾ Verhältnis Lactat/Pyruvat zum Zeitpunkt t1 ⁴⁾ Lactat (mmol/l) zum Zeitpunkt t2 = nach 16 Tagen Einnahmedauer ⁵⁾ Pyruvat (mmol/l) zum Zeitpunkt t2 = nach 16 Tagen Einnahmedauer ⁶⁾ Verhältnis Lactat/Pyruvat zum Zeitpunkt t2 ⁷⁾ Differenz L7P (t2) - L/P (t1) | | | | | | | | | |

Es hat sich überraschend gezeigt, dass die Pyruvat-Konzentration signifikant angestiegen ist über den Behandlungszeitraum.

Dies indiziert eine gesteigerte Leistungsfähigkeit der Mitochondrien und somit der gesamten Physiologie des Körpers.

Die erfindungsgemäßen Emulsionen kommen daher hervorragend zur Behandlung und Prophylaxe der mitochondrialen Dysfunktion und der damit im Zusammenhang stehenden Erkrankungen zum Einsatz.

## Patentansprüche

1. Flüssige wässrige Emulsion umfassend
a) ein oder mehrere Phospholipid(e),
b) ein oder mehrere Polyole ausgewählt aus der Gruppe bestehend aus Glycerin und/oder Saccharid(en), und
c) lipophile Komponenten umfassend mittelkettige Triglyceride und Ubichinon-10,
wobei das Gewichtsverhältnis von Phospholipid zum Gesamtgewicht der lipophilen Komponenten 1:0,1 bis 1:0,98 beträgt.

2. Flüssige wässrige Emulsion gemäß Anspruch 1, **dadurch gekennzeichnet, dass** das Gesamtgewicht von Phospholipid und lipophilen Komponenten 5 bis 30 Gew.-%, bezogen auf das Gesamtgewicht der Emulsion, ist.

3. Flüssige wässrige Emulsion gemäß Anspruch 1 oder 2, **dadurch gekennzeichnet, dass** die lipophilen Komponenten zusätzlich ausgewählt sind aus der Gruppe bestehend aus Fettsäuretriglyceride, die keine mittelkettigen Triglyceride sind, Vitamin E, Vitamin D, Vitamin K, Vitamin A, Liponsäure, Carotinoide, Tocopherole, Tocotrienole und beliebige Mischungen hiervon.

4. Flüssige wässrige Emulsion gemäß einem oder mehreren der vorangehenden Ansprüche, **dadurch gekennzeichnet, dass** die Emulsion Citronensäure und/oder Alkali- und/oder Erdalkalicitrate aufweist.

5. Flüssige wässrige Emulsion gemäß Anspruch 4, **dadurch gekennzeichnet, dass** die Emulsion 0,1 bis 5 Gew.-% Citroensäure und/oder Citrate enthält.

6. Flüssige wässrige Emulsion gemäß einem oder mehreren der vorangehenden Ansprüche, **dadurch gekennzeichnet, dass** die Emulsion 30 bis 70 Gew.-% Polyol(e) aufweist.

7. Flüssige wässrige Emulsion gemäß einem oder mehreren der vorangehenden Ansprüche, **dadurch gekennzeichnet, dass** die Emulsion zusätzlich Ascorbinsäure enthält.

8. Flüssige wässrige Emulsion gemäß Anspruch 7, **dadurch gekennzeichnet, dass** die Emulsion 0,1 bis 5 Gew.-% Ascorbinsäure aufweist.

9. Verwendung der flüssigen wässrigen Emulsion gemäß einem oder mehreren der vorangehenden Ansprüche als Getränk, insbesondere als Diätetikum.

10. Pulver erhältlich aus einer flüssigen wässrigen Emulsion gemäß einem oder mehreren der Ansprüche 1 bis 8 durch Trocknung, insbesondere Gefriertrocknung oder Sprühtrocknung.

11. Pharmazeutische Zubereitung umfassen die flüssige wässrige Emulsion gemäß einem oder mehreren der Ansprüche 1 bis 8 oder ein Pulver gemäß Anspruch 10.

12. Pharmazeutische Zubereitung gemäß Anspruch 11 zur Verwendung in der Therapie und/oder Prophylaxe mitochondrialer Dysfunktion bei Mensch oder Tier, insbesondere Säugetier.

13. Pharmazeutische Zubereitung gemäß Anspruch 11 oder 12 zur Behandlung und/oder Prophylaxe von Erkrankungen, ausgewählt aus der Gruppe Typ II Diabetes, Arteriosklerose, Kardiomyopathie, Fibromyalgie, Alzheimersche Krankheit, Parkinsonsche Krankheit, Adipositas, Hypertonie und chronisches Müdigkeitssyndrom (CFS, chronical fatigue syndrome).
